**Europäisches Patentamt**

**European Patent Office**

**Office européen des brevets**

(11) Veröffentlichungsnummer : **0 368 216 B1**

(12) **EUROPÄISCHE PATENTSCHRIFT**

(45) Veröffentlichungstag der Patentschrift :
05.08.92 Patentblatt 92/32

(21) Anmeldenummer : **89120524.7**

(22) Anmeldetag : **06.11.89**

(51) Int. Cl.⁵ : **A61K 9/24, A61K 9/22**

(54) **Feste pharmazeutische Retardform.**

(30) Priorität : **10.11.88 DE 3838094**

(43) Veröffentlichungstag der Anmeldung :
**16.05.90 Patentblatt 90/20**

(45) Bekanntmachung des Hinweises auf die
Patenterteilung :
**05.08.92 Patentblatt 92/32**

(84) Benannte Vertragsstaaten :
**AT BE CH DE FR GB IT LI NL SE**

(56) Entgegenhaltungen :
**EP-A- 0 052 075**
**EP-A- 0 052 075**
**DE-B- 1 492 140**
**GB-A- 1 146 621**
**US-A- 3 939 259**
**US-A- 4 289 795**
**US-A- 4 755 387**

(56) Entgegenhaltungen :
**PATENT ABSTRACTS OF JAPAN, unexamined
applications, Field C, Band 8, Nr. 178, 16.
August 1984 THE PATENT OFFICE JAPANE-
SEGOVERNMENT, Seite 25 C 238**
**PATENT ABSTRACTS OF JAPAN, unexamined
applications, Sektion C, Band 3, Nr. 138, 16.
November 1979 THE PATENT OFFICE JAPA-
NESEGOVERNMENT, Seite 159 C 64**

(73) Patentinhaber : **Nordmark Arzneimittel GmbH.**
**Postfach 44**
**W-2082 Uetersen 1 (DE)**

(72) Erfinder : **Moest, Thomas,Dr.**
**An der Duene 9**
**W-2082 Moorrege (DE)**

(74) Vertreter : **Karau, Wolfgang, Dr. et al**
**BASF Aktiengesellschaft Carl-Bosch-Strasse
38**
**W-6700 Ludwigshafen (DE)**

## Beschreibung

Die Erfindung betrifft eine feste oral applizierbare Arzneiform mit verzögerter Wirkstoff-Freigabe aus einem wirkstoffhaltigen Kern und einem Überzug, der aus einer fett- oder wachsartigen hydrophoben Substanz besteht, die ein wasserunlösliches Polymeres zur Regelung der Wirkstoff-Freigabe und ganz außen ein Gleitmittel (= Antiadhäsivum) enthält, sowie ein Verfahren zur Herstellung derselben.

Zur Herstellung von festen Formen wie Tabletten, Granulaten oder Pellets mit verzögerter Freisetzung wird häufig das Coatingverfahren eingesetzt. Für Retardformen ergibt ein Überzug mit einer diffusionskontrollierenden Hülle den Vorteil einer angenähert linearen Freisetzung, d.h. eine Freisetzung mit einer Kinetik O. Ordnung, wobei in jedem Zeitintervall gleiche Wirkstoffmengen freigesetzt werden.

US 4 755 387 beschreibt die Herstellung magensaftresistenter, aber darmsaftlöslicher, also nicht retardierender Beschichtungen aus 2 verschiedenen Lipiden, die theoretisch auch wasserlösliche oder unlösliche Polymere zugemischt enthalten können.

In der EP-A 52 075 wird das Retardieren durch überziehen mit einer Mischung zweier wasserunlöslicher Polymerer beschrieben.

Für das Überziehen werden meistens Lacklösungen von wasserunlöslichen Polymeren in organischen Lösungsmitteln eingesetzt. Aus Gründen der Umweltbelastung, wegen toxischer Eigenschaften sowie der Feuergefahr sind organische Lösungen problematisch zu handhaben. Beim Ersatz durch wäßrige Dispersionen dieses Polymeren entfällt zwar das organische Lösungsmittel, aber dafür treten andere Nachteile auf:

Die Systeme sind empfindlich gegen Kälte und mikrobiologische Verunreinigungen. Handhabung, Transport und Lagerung großer Mengen von Dispersionen mit dem Hauptbestandteil Wasser sind aufwendig. Deutlich erhöhter Mengenbedarf des Polymeren auf das ca. 3-fache, da die Homogenität des Lackauftrages schlechter ist als bei organischen Lösungen. Aufgrund der höheren Schichtdicke verlängerte Lackierzeiten, woraus letztlich eine Reduktion der Produktionskapazität resuliert. Erhöhte Anforderungen an die Zusatzstoffe, insbesondere die Weichmacher, um ein "Verfließen" der Latexteilchen beim Auftragen sicherzustellen. Höhere Kosten des Polymeren um den Faktor 2 bis 6, zurückgerechnet auf den Trockengehalt für die Dispersionsformen, im Vergleich zu den Reinsubstanzen. Diese erhöhten Polymerkosten in Verbindung mit dem erhöhten Mengenbedarf und den daraus resultierenden zusätzlich erhöhten Lackierkosten lassen die wäßrige Lackierung trotz Einsparung der organischen Lösungsmittel unwirtschaftlich erscheinen.

Um diese Nachteile zu mindern, müßte die Menge des Polymeren reduziert werden. Dies würde jedoch zu einer nicht zulässigen Beschleunigung der Freisetzung des Arzneistoffes führen.

Der Erfindung lag daher die Aufgabe zugrund, dem geschilderten Mißstand abzuhelfen und ein einfaches, rasch durchführbares und wirtschaftliches Verfahren zum Retard-Beschichten mit guter Steuerbarkeit der Wirkstoff-Freigabe zu entwickeln, das außerdem möglichst geringe Überzugsmengen erfordert.

Die Lösung dieser Aufgabe besteht in den Retardformen nach den Ansprüchen 1 und 2 und in dem Verfahren zu deren Herstellung nach Anspruch 3.

Der wirkstoffhaltige Kern kann aus einer Tablette, einem Pellet oder einem Granulatkorn bestehen.

Der Begriff "pharmazeutische Retardform" ist dem Fachmann geläufig und bedarf keiner Erläuterung.

Unter pharmazeutischen Wirkstoffen im Sinne der Erfindung sind alle Stoffe mit einer pharmazeutischen Wirkung und möglichst geringen Nebenwirkungen zu verstehen. Die Wirkstoffmenge pro Dosiseinheit kann je nach Wirksamkeit und Freisetzungsgeschwindigkeit in weiten Grenzen variieren. Die einzige Bedingung ist, daß sie zur Erzielung der gewünschten Wirkung ausreicht. Es kommen praktisch alle pharmazeutischen Wirkstoffe in Betracht, die sich im Verdauungstrakt lösen.

Als übliche galenische Hilfsstoffe zur Bildung des Granulat-, Tabletten- oder Pelletkerns kommen vor allem Bindemittel für den Wirkstoff in Betracht, beispielsweise Cellulosederivate, Polyvinylpyrrolidon oder Gelatine, ferner z.B. inerte Verdünnungsmittel wie Dextrose, Zucker, Sorbit, Mannit.

Der hydrophobe Überzug des wirkstoffhaltigen Kerns soll im wesentlichen aus einem physiologisch unbedenklichen Fett oder Wachs bestehen. Physiologisch unbedenklich heißt mit anderen Worten ungiftig. Die Fette werden verdaut, die Wachse in der Regel unverändert ausgeschieden. Beispiele für geeignete Wachse sind: Carnauba-Wachs, Ester der Montansäure, Bienenwachs, Cetylpalmitat. Beispiele für geeignete Fette sind Glycerintristearat und Glycerintribehenat.

Sie werden in fein zerkleinerter Form mit einem mittleren Teilchendurchmesser von 1 - 100 μm, wobei nicht mehr als 10 Gew.-% Teilchendurchmesser von mehr als 100 μm besitzen, in Wasser dispergiert.

Für den Überzug können als übliche galenische Hilfsstoffe vor allem Dispergierhilfsmittel wie Polyvinylpyrrolidon, oxethylierte Sorbitan-Fettsäureester, oxethyliertes hydriertes Ricinusöl, Triglyceride von $C_8$-$C_{10}$-Fettsäuren, Weichmacher wie Triethylcitrat, Dibutylphthalat, hydriertes tierisches Fett, acetylierte Fettsäuremonoglyceride, Farbstoffe und gegebenenfalls Geschmackstoffe eingesetzt werden.

Erfindungsgemäß enthalten die Überzüge 1/10 bis 3 Teile, vorzugsweise 1/5 bis 2, insbesondere 1/3 bis 1 Teil eines wasserunlöslichen Polymeren, bezogen auf 1 Teil fett- oder wachsartiger hydrophober Beschichtungsmasse. Als Polymere kommen alle in Betracht, die zur Herstellung von Retardüberzügen üblich sind, z.B. Ethylcellulose, Copolymerisate von Ethylacrylat und Methylmethacrylat oder von Ethylacrylat, Methylmethacrylat und Methacrylsäure-2-trimethylammoniumethylester-hydrochlorid.

Das Polymere in dem hydrophoben Überzug hat zwei Funktionen: zum einen wirkt es als Bindemittel für die Fett- oder Wachspartikel untereinander sowie zwischen ihnen und der Oberfläche des Tablettenkerns, und zum anderen reguliert es die Wirkstoff-Freigabe durch die hydrophobe Schicht hindurch. Beide Funktionen waren nicht vorherzusehen.

Durch die Wahl der geeigneten Art und Menge des Polymeren und der Dicke der hydrophoben Schicht, jeweils abgestimmt auf den Wirkstoff, kann praktisch jede gewünschte Freigabe-Geschwindigkeit eingestellt werden.

Als äußerste Schicht wird ein Antiadhäsivum wie Talkum, Magnesiumstearat oder hochdisperse Kieselsäure mit einer wäßrigen Dispersion eines wasserunlöslichen Polymeren wie Ethylcellulose oder einer wäßrigen Lösung eines wasserlöslichen Polymeren wie Methylcellulose, Hydroxypropylcellulose, Hydroxypropyl-methylcellulose oder Polyvinylpyrrolidon als Bindemittel aufgetragen. Die Menge des Antiadhäsivums liegt im Bereich 0,1 bis 5 %, vorzugsweise im Bereich 0,5 bis 2 %, bezogen auf die eingesetzte Kernmenge. Der Polymerenanteil dieser äußersten Schicht liegt im Bereich von 20 bis 400, vorzugsweise 50 bis 100 Gew.-%, bezogen auf die Menge des Antiadhäsivums.

Nach der Beschichtung werden die umhüllten Arzneiformen über die Schmelztemperatur der hydrophoben fett- oder wachsartigen Beschichtungssubstanz, d.h. auf eine Temperatur von 30 bis 120, vorzugsweise 40 bis 100, insbesondere 50 bis 90°C erwärmt.

Das Beschichten und Erwärmen erfolgt jeweils in bewegtem Zustand, beispielsweise in einem Wirbelschichtgerät, einem Lochtrommelcoater oder Dragierkessel.

Es ist überraschend, daß feste Arzneiformen wie Tabletten, Granulate oder Pellets mit einer hydrophoben Beschichtung fett- bzw. wachsartiger Substanzen gleiches lineares Freisetzungsverhalten wie polymerbeschichtete Formen aufweisen, wenn in der hydrophoben Beschichtung ein Anteil eines wasserunlöslichen Polymeren enthalten ist.

Ebenso überraschend ist es, daß sich eine fett- oder wachsähnliche Schicht auf festen Arzneiformen aus einer wäßrigen, feinverteilten Suspension der hydrophoben Substanz auftragen läßt, wenn ein wasserunlösliches Polymeres -gegebenenfalls in nur geringen Anteilen - als Bindemittel für die hydrophoben Partikeln eingesetzt wird.

Zwar werden auch beim erfindungsgemäßen Verfahren wäßrige Dispersionen eingesetzt, aber die erfoderlichen Mengen und damit Schichtdicken auf den wirkstoffhaltigen Kernen sind im Vergleich zur Beschichtung nur mit Polymeren gering, wodurch nicht nur die Produktion beschleunigt, sondern auch die Wirtschaftlichkeit erhöht wird.

Beispiele

1. Retardierte Kaliumchloridkristalle

A) Zusammensetzung pro Amsatz

| | |
|---|---:|
| Kaliumchloridkristalle 0,50-1,00 mm | 3.560 g |
| Ester der Montansäure (Hoechstwachs®E) | 140 g |
| 30 %ige wäßrige Ethylcellulose-Dispersion (Aquacoat®ECD 30) Trockensubstanz | 70 g |
| Acetylierte Mono- und Diglyceride (Myvacet®9-45) | 15 g |
| Monooleat von 20fach oxethyliertem Sorbitan (Tween®80) | 1 g |
| Hydroxypropylmethylcellulose | 25 g |
| Talkum | 50 g |
| Siliciumdioxid, hochdispers | 9 g |
| | 3.870 g |

B) Herstellung

Die Kaliumchloridkristalle wurden zunächst mit einer Suspension des feingemahlenen Wachses und 20 g Talkum in der mit Tween 80 versehenen Latexdispersion Aquacoat ECD 30, wobei die Gesamtkonzentration der Dispersion 25 % betrug, in einem Wirbelschichtcoater bei einer Zulufttemperatur von 50°C überzogen. Anschließend erfolgte das Auftragen der Antiadhäsionsschicht durch Aufsprühen einer Suspension des restlichen Talkums und des hochdispersen Siliciumdioxids in einer 5 %igen Hydroxypropylmethylcelluloselösung in Wasser. Zum Schluß wurden die überzogenen Kristalle 30 min unter Wirbeln auf 80°C erwärmt.

Die KCl-Freisetzung des Endproduktes ist in weitem Bereich streng linear. Mit 90 % freigesetztem Kaliumchlorid nach 7 h entspricht es in der Freisetzungscharakteristik weitgehend vergleichsweise mit 3 % Ethylcellulose aus ethanolischer Lösung überzogenem Kaliumchlorid. Das erfindungsgemäße Verfahren ist jedoch einfacher und wirtschaftlicher.

Vergleichsversuch

Zum Vergleich wäßrig überzogene Kaliumchloridkristalle mit einem Überzug aus Aquacoat ECD 30 und Myvacet 9-45 (25 %) unter Talkumzusatz mit einer Gesamtkonzentration der Dispersion von 25% erfordern für eine vergleichbare Freisetzung mit 90 % nach 7 h eine relative Trockenmasse an Aquacoat von 10 %, also ca. 2,5 mal so viel wie erfindungsgemäß. Die Dauer des Lackierprozesses war unter optimierten Bedingungen im Vergleich zum erfindungsgemäßen Verfahren ca. 20 % höher.

2. Retardierte Theophyllinpellets

A) Zusammentsetzung pro Ansatz

```
Theophyllin mikronisiert (90 % <100 µm)         2 625,00 g
Microkristalline Cellulose (Avicel® PH 102)       700,00 g
Polyvinylpyrrolidon (Kollidon® 25)                175,00 g
30 % wäßrige Dispersion von Acryl- und
Methacrylsäureester-Copolymerisaten mit
Trimethylammoniummethacrylat
(Eudragit® RS 30 D) Trockensubstanz                60,00 g
Glycerintristearat                                100,00 g
Dibutylphthalat                                    15,00 g
(Cremophor® RH 40)                                  2,00 g
Methylcellulose (Methocel® MC)                     40,00 g
Talkum                                             40,00 g
                                                 3 757,00 g
```

B. Herstellung

Theophyllin-Pulver wurde mit mikrokristalliner Cellulose und Polyvinylpyrrolidon in einem Pharma-Pflugscharmisch- und -granuliergerät vermischt und mit Wasser befeuchtet. Das sich ausbildende pelletförmige Granulat wurde getrocknet und die Fraktion 1 bis 2 mm herausgesiebt.

3 500 g dieser Pellets wurden zunächst mit einer Suspension des feingemahlenen Wachses und 40 % der Talkummenge in der mit Cremophor RH 40 versehenen Latexdispersion Eudragit RS 30, wobei die Gesamtkonzentration der Dispersion 25 % betrug, in einem Wirbelschichtcoater bei einer Zulufttemperatur von 55°C überzogen. Anschließend erfolgte das Auftragen der Antiadhäsionsschicht durch Aufsprühen einer Suspension von 60 % der Talkummenge in einer 3%igen Methocellösung in Wasser. Die überzogenen Pellets wurden abschließend unter Wirbeln 30 min. auf 60°C erwärmt.

Wäßrig überzogene Theophyllinpellets der gleichen Charge mit einem Überzug aus Eudragit® RS 30 D, Dibutylphthalat (25 %) und Talkum (30 %, wobei die Prozentangaben sich jeweils auf die Eudragit-Trockenmasse beziehen) mit einer Gesamtkonzentration der Dispersion von 25 % erforderten für eine vergleichbare Freisetzung mit 90 % nach 10 h eine relative Trockenmasse an Eudragit von 6 %, also 3,5 mal so viel wie erfindungsgemäß. Die Dauer des Lackierprozesses war trotz optimierter Bedingungen im Vergleich zum erfindungsgemäßen Verfahren ca. 15 % höher.

## Patentansprüche

1. Feste pharmazeutische Retardform, bestehend aus einem den Wirkstoff neben üblichen galenischen Hilfsstoffen enthaltenden Kern, einem die Freisetzung des Wirkstoffs verzögernden Überzug sowie einer antiadhäsiven äußeren Beschichtung, dadurch gekennzeichnet, daß der Überzug aus einer physiologisch unbedenklichen, im Bereich von 30 bis 120°C schmelzenden Fett- oder Wachsschicht besteht, die neben üblichen galenischen Hilfsstoffen Anteile mindestens eines wasserunlöslichen Polymeren enthält.

2. Feste Arzneiform nach Anspruch 1, dadurch gekennzeichnet, daß das Gewichtsverhältnis von wasserunlöslichen Polymeren zur hydrophoben Schichtmasse ohne das Polymere und ohne die antiadhäsive äußere Beschichtung im Bereich von 1:10 bis 3:1 liegt.

3. Verfahren zur Herstellung fester pharmazeutischer Formen nach Anspruch 1 oder 2, dadurch gekennzeichnet, daß eine Mischung je einer wäßrigen Dispersion der kleinteiligen hydrophoben Substanz und des Polymeren auf die wirkstoffhaltigen Kerne nach üblichen Methoden aufgetragen wird, die so beschichteten Kerne nach üblichen Methoden getrocknet und mit einer Mischung aus Gleitmittel und polymerem Bindemittel beschichtet und unter ständiger Bewegung über die Schmelztemperatur des Fettes oder Wachses erwärmt und wieder auf Raumtemperatur abgekühlt werden.

## Claims

1. A solid pharmaceutical sustained-release form, consisting of a core containing the active compound as well as conventional pharmaceutical auxiliaries, a coat which delays the release of the active compound and an antiadhesive outer layer, wherein the coat consists of a physiologically acceptable layer of fat or wax which melts in the range from 30 to 120°C and contains, in addition to conventional pharmaceutical auxiliaries, one or more water-insoluble polymers.

2. A solid drug form as claimed in claim 1, wherein the weight ratio of water-insoluble polymer to hydrophobic layer material without the polymer and without the antiadhesive outer layer is from 1 : 10 to 3 : 1.

3. A process for the preparation of a solid pharmaceutical form as claimed in claim 1 or 2, wherein a mixture of an aqueous dispersion of a finely divided hydrophobic substance and an aqueous dispersion of the polymer is applied to the active compound-containing cores by a conventional method, the cores coated in this manner are dried and coated with a mixture of lubricant and polymeric binder by a conventional method and are heated to above the melting point of the fat or wax with constant movement and are cooled again to room temperature.

## Revendications

1. Formes pharmaceutiques solides à retard consistant en un noyau contenant la substance active avec des produits auxiliaires galéniques usuels, un revêtement retardant la libération de la substance active et un enduit extérieur anti-adhésif, caractérisées en ce que le revêtement consiste en une couche de matière grasse ou de cire acceptable pour l'usage pharmaceutique, fondant dans l'intervalle de 30 à 120 degrés C, qui, avec des produits auxiliaires galéniques usuels, contient au moins un polymère insoluble dans l'eau.

2. Forme pharmaceutique solide selon revendication 1, caractérisée en ce que les proportions relatives en poids entre le polymère insoluble dans l'eau et la masse de revêtement hydrophobe sans le polymère et sans l'enduit extérieur anti-adhésif se situent dans l'intervalle de 1 : 10 à 3 : 1.

3. Procédé pour préparer des formes pharmaceutiques solides selon revendication 1 ou 2, caractérisé en ce que l'on applique par des techniques usuelles un mélange d'une dispersion aqueuse de la substance hydrophobe en fines particules et d'une dispersion aqueuse du polymère sur les noyaux contenant la substance active, on sèche les noyaux ainsi revêtus par des techniques usuelles et on les enduit d'un mélange d'un agent lubrifiant et d'un liant polymère puis on les chauffe en les remuant en permanence au-dessus de la température de fusion de la matière grasse ou cire et on les refroidit à nouveau à température ambiante.